# EUROPEAN PATENT APPLICATION

(11) **EP 3 692 837 A1**
(43) Date of publication of application: **12.08.2020**
(21) Application number: 19155875.8
(22) Date of filing: 07.02.2019
(51) Int. Cl.: A24F 47/00, A61M 11/04, A61M 15/06, A61M 15/00, A61M 11/00, A61M 16/00

(54) **FLAVOUR DELIVERY ARTICLE, SMOKING SUBSTITUTE APPARATUS AND SMOKE SUBSTITUTE DEVICE**

(71) Applicant: NERUDIA LIMITED, Liverpool L24 9HP (GB)
(72) Inventor: The designation of the inventor has not yet been filed
(74) Representative: Mewburn Ellis LLP

(57) **Abstract**

A flavour delivery article (210) for use with a smoking substitute device (101). The flavour delivery article contains a fragranced fluid (212), and is configured to release a mist of the fragranced fluid upon actuation of the flavour delivery article for olfactory flavour delivery.

## Description

### Field of the Invention

The present invention relates to a smoking substitute apparatus and, in particular, a smoking substitute apparatus that is able to deliver flavour to a user.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, e-liquid is heated by a heating device to produce an aerosol vapour which is inhaled by a user. Many e-cigarettes also deliver flavour to the user to enhance the experience. In such e-cigarettes, flavour compounds are contained in the e-liquid that is heated. However, toxicology restrictions are placed on the amount of flavour that can be contained in the e-liquid, and this can result in some e-liquid flavours delivering a weak and underwhelming taste sensation to consumers in the pursuit of safety. Further, there is a view that providing a flavourant as part of the e-liquid, such that the flavourant is vaporised with the e-liquid, may be disadvantageous.

There may be a need for improved design of smoking substitute systems, in particular in regards to the delivery of flavour to a user.

The present disclosure has been devised in the light of the above considerations.

### Summary of the Invention

At its most general, the present invention relates to the delivery of flavour to a user by olfactory flavour delivery.

According to a first aspect, there is provided a flavour delivery article for use with a smoking substitute apparatus, wherein the flavour delivery article contains a fragranced fluid, and is configured to release a mist of the fragranced fluid upon actuation of the flavour delivery article for olfactory flavour delivery.

Advantageously, flavour can be sensed through the nose of a user compared to within the mouth of the user. Specifically, the effect of flavour can be provided through the sense of smell rather than the sense of taste. In this way, the delivery of flavour is separate from delivery of e-liquid vapour, which may or may not contain nicotine. This can affect and/or increase the amount and/or strength of flavour sensed by a user. Furthermore, as the mist (i.e. spray) of fragranced fluid is released upon actuation of the flavour delivery article, a user can choose whether they want flavour delivery during use of the smoking substitute device (i.e. during vaping).

Preferably, the flavour delivery article is configured to release a mist of the fragranced fluid upon actuation (i.e. activation) of the flavour delivery article by a user of the smoking substitute device.

Optionally, the flavour delivery article may be configured to release the mist of the fragranced fluid only when the flavour delivery article is actuated, and may be configured to not release the mist of the fragranced fluid when the flavour delivery article is not actuated.

In this way, a user can choose to activate flavour delivery or to deactivate flavour delivery during vaping depending on whether or not they would like to receive flavour during each use (i.e. each puff) of the smoking substitute device.

Conveniently, the flavour delivery article may be a compressible pocket. The compressible pocket may be biased into an uncompressed state, and configured to release the mist of the fragranced fluid upon compression of the compressible pocket.

The compressible pocket provides a user-friendly and simple way of actuating the flavour delivery article in order to deliver flavour. A user can easily compress the pocket like a blower to push a mist of the fragranced fluid, which is contained in the compressible pocket, into the surrounding ambient air. The mist of the fragranced fluid can then be received by the user by breathing in the mist of the fragranced fluid through the nose.

The compressible pocket may comprise a valve inlet for introducing ambient air into the flavour delivery article.

In this way, after compression of the pocket to release the mist of fragranced fluid, air can be reintroduced via the valve inlet into the compressible pocket to enable further and ongoing compressions of the pocket. This valve inlet can help to prevent the formation of vacuum-like conditions in the pocket, which would prevent the pocket from being reusable. In other words, the valve inlet allows the pocket to refill with ambient air after each compression, such that the pocket can be actuated repeatedly. Thus, the release of the mist of fragranced fluid for olfactory flavour delivery can be repeated.

Optionally, the valve inlet may be a one-way valve which allows the flow of air through the valve inlet into the flavour delivery article, but prevents the flow of air through the valve inlet out of the flavour delivery article.

Accordingly, air can be allowed back into the pocket to enable ongoing compressions of the pocket by a user, and the air does not escape from the pocket via the valve inlet. This configuration helps to bias the pocket into an uncompressed state.

The flavour delivery article may comprise an outlet mister for releasing the mist of the fragranced fluid upon actuation of the flavour delivery article.

The outlet mister may release the mist of fragranced fluid in a specified particle size, such that the fragranced fluid can be easily and efficiently received by a user's nose for olfactory flavour delivery.

Optionally, the outlet mister may comprise a plurality of holes or slits for releasing the mist of the fragranced fluid.

The holes or slits may be sized to release a preferred and/or specified particle size of the mist of fragranced fluid.

Conveniently, the outlet mister may be a silicone valve.

When the flavour delivery apparatus comprises both an outlet mister and a valve inlet, the outlet mister and valve inlet may be integral with one another to form a single combined valve. Upon actuation of the flavour delivery apparatus, the single combined valve is configured to act as an outlet mister for releasing the mist of the fragranced fluid. When the flavour delivery apparatus is not actuated (e.g. after actuation of the flavour delivery apparatus), the single combined valve is configured to act as the valve inlet to allow the flow of air through the single combined valve into the pocket. This enables repeatable compressions of the pocket.

Advantageously, the fragranced fluid may be an oil-based liquid and/or a sugar-based liquid.

According to a second aspect, there is provided a smoking substitute apparatus for a smoking substitute device, the smoking substitute apparatus comprising a tank for containing a vaporisable e-liquid, a heating element for heating the vaporisable e-liquid, and the flavour delivery article according to the first aspect.

The smoking substitute apparatus may be a consumable.

Advantageously, flavour can be sensed through the nose rather than through the mouth of a user. The delivery of the vaporised e-liquid to the user is via the mouth, whereas the delivery of flavour to the user can be through the nose, via an olfactory flavour delivery mechanism. Therefore, the delivery of the e-liquid which may or may not contain nicotine is separate from the delivery of the flavour. This can affect and/or increase the amount and/or strength of flavour sensed by a user. Furthermore, as the mist of fragranced fluid is released upon actuation of the flavour delivery article, a user can choose whether they want flavour delivery in each use (i.e. each puff) of the smoking substitute device (i.e. during vaping) by actuating the flavour delivery article.

Optionally, the smoking substitute apparatus may further comprise an outer casing, and the flavour delivery article may be moulded into the outer casing.

Accordingly, the flavour delivery article is positioned where a user may easily activate it (e.g. by pressing using a finger or otherwise the flavour delivery article), because the flavour delivery article is positioned on an outside of the smoking substitute device. Thus, the flavour delivery article can be easily operated to release a flavour during vaping, under the control of a user of the smoking substitute device.

One end of the smoking substitute apparatus may define a mouthpiece. When the flavour delivery article comprises an outlet mister for releasing the mist of the fragranced fluid upon actuation of the flavour delivery article, the outlet mister may be positioned so that it is closer to the end of the smoking substitute apparatus comprising the mouthpiece than it is to the other end of the smoking substitute apparatus.

In this way, the release of the mist of fragrance fluid occurs closer to or proximate the mouthpiece, and thus closer to a user of the smoking substitute device. The mist of fragranced fluid is thereby released in the region of a user's nose for olfactory flavour delivery.

The outlet mister may be configured to release the mist of the fragranced fluid generally towards the mouthpiece. Additionally, the outlet mister may be positioned, arranged and/or orientated such that, in use, the mist of fragranced fluid is released towards the nose of a user. For example, the outlet mister may be positioned such that in use of the smoking substitute device, the outlet mister is directly underneath or adjacent to a nose of a user.

By releasing the mist of the fragranced fluid towards the mouthpiece, the mist of fragranced fluid is directed towards a user's nose during vaping. In this way, the user can receive a stronger flavour as more of the released mist of fragranced fluid can be received by the user' nose.

Preferably, the flavour delivery article is separated from the tank for containing the e-liquid.

In this way, the fragranced liquid in the flavour delivery article is separate to the e-liquid and is therefore not in the primary air flow of the vaporised e-liquid through the mouthpiece. The fragranced fluid is thus not mixed or contaminated with the e-liquid during use, which may or may not contain nicotine. According to a third aspect of the invention, there is provided an electronic cigarette device comprising the smoking substitute apparatus according to the second aspect of the invention, and a main body comprising a power source for providing power to the heating element of the smoking substitute apparatus.

The fragranced fluid may comprise a flavourant. The term "flavourant" is used herein to describe a compound or combination of compounds that provide flavour and/or aroma. For example, the flavourant may be configured to interact with a sensory receptor of a user (such as an olfactory or taste receptor). The flavourant may include one or more volatile substances.

The flavourant may be provided in solid, gel or liquid form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The smoking substitute apparatus may be in the form of a consumable. The consumable may be configured for engagement with a main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol former (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol former (e.g. e-liquid) of the system may be replenished by re-filling e.g. a reservoir of the smoking substitute apparatus with the aerosol former (rather than replacing a consumable component of the apparatus).

In light of this, it should be appreciated that some of the features described herein as being part of the smoking substitute apparatus may alternatively form part of a main body for engagement with the smoking substitute apparatus (i.e. when the smoking substitute apparatus is in the form of a consumable).

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a reservoir configured to store an aerosol former, such as an e-liquid. The e-liquid may, for example, comprise a base liquid and e.g. nicotine. The base liquid may include propylene glycol and/or vegetable glycerine. The e-liquid may be flavourless. That is, the e-liquid may not contain any flavourants and may consist solely of a base liquid of propylene glycol and/or vegetable glycerine and nicotine.

The reservoir may be in the form of a tank. At least a portion of the tank may be translucent. For example, the tank may comprise a window to allow a user to visually assess the quantity of e-liquid in the tank. A housing of the smoking substitute apparatus may comprise a corresponding aperture (or slot) or window that may be aligned with a translucent portion (e.g. window) of the tank. The reservoir may be referred to as a "clearomizer" if it includes a window, or a "cartomizer" if it does not.

The smoking substitute apparatus may comprise a passage for fluid flow therethrough. The passage may extend through (at least a portion of) the smoking substitute apparatus, between openings that may define an inlet and an outlet of the passage. The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage. In this respect, the tank may surround the passage.

The smoking substitute apparatus may comprise an aerosol-generator. The aerosol generator may comprise a wick. The aerosol generator may further comprise a heater. The wick may comprise a porous material. A portion of the wick may be exposed to fluid flow in the passage. The wick may also comprise one or more portions in contact with liquid stored in the reservoir. For example, opposing ends of the wick may protrude into the reservoir and a central portion (between the ends) may extend across the passage so as to be exposed to fluid flow in the passage. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. This vapour may subsequently cool to form an aerosol in the passage.

The smoking substitute apparatus (or main body engaged with the smoking substitute apparatus) may comprise a power source. The power source may be electrically connected (or connectable) to a heater of the smoking substitute apparatus (e.g. when engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface may be configured to transfer electrical power from the power source to a heater of the consumable.

The electrical interface may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may form part of the consumable or the main body.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1A is a front view of a smoking substitute system in an engaged position;
Figure 1B is a front view of the smoking substitute system of Figure 1A in a disengaged position;
Figure 1C is a section view of a smoking substitute apparatus;
Figure 2 is a section view of a smoking substitute apparatus having a flavour delivery apparatus according to an embodiment; and
Figure 3 is an enlarged section view of a flavour delivery apparatus according to an embodiment.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

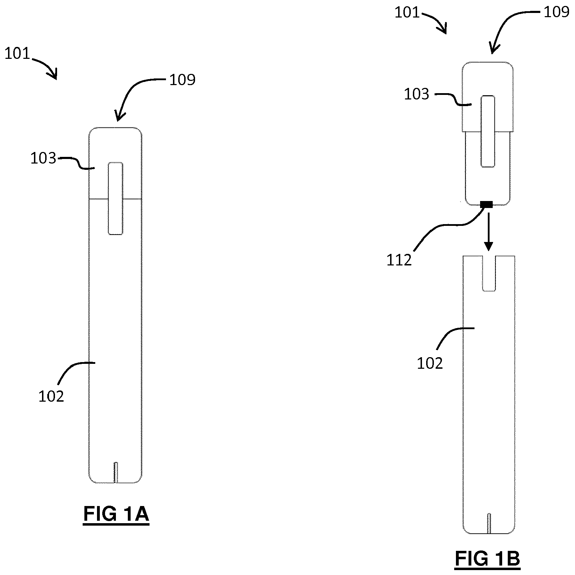

Figures 1A and 1B illustrate a smoking substitute system in the form of an e-cigarette system 101. The system 101 comprises an e-cigarette device defining a main body 102 of the system 101, and a smoking substitute apparatus in the form of an e-cigarette consumable (or "pod") 103. In the illustrated embodiment the consumable 103 (smoking substitute apparatus) is removable from the main body (e-cigarette device), so as to be a replaceable component of the system 101. In other words, the e-cigarette system 101 is a closed system.

As is apparent from Figures 1A and 1B, the consumable 103 is configured to engage the main body 102. Figure 1A shows the main body 102 and the consumable 103 in an engaged state, whilst Figure 1B shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol-former, which in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless (i.e. it does not include any added flavourant). That is, if the e-liquid 104 were to be inhaled (i.e. in aerosol form) by a user, it would not have a particularly perceptible flavour or taste.

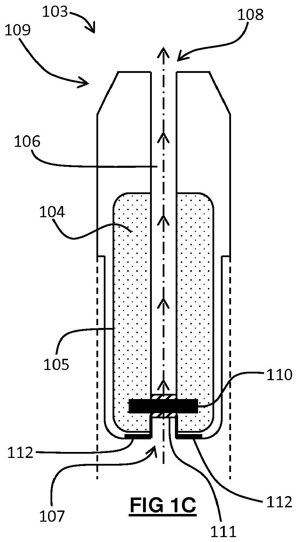

As is more apparent from Figure 1C, this e-liquid 104 is stored within a reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol former) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a passage 106 that extends between an inlet 107 and an outlet 108 at opposing ends of the consumable 103. In this respect, the passage comprises an upstream end at the end of the consumable 103 that engages with the main body 102, and a downstream end at an opposing end of the consumable 103 that comprises a mouthpiece 109 of the system 101. When the consumable 103 is engaged with the main body 102, a user can inhale (i.e. take a puff) via the mouthpiece 109 so as to draw air through the passage 106, and so as to form an airflow (indicated by arrows) in a direction from the inlet 107 to the outlet 108 of the passage 106. Although not illustrated, the passage 106 may be partially defined by a tube (e.g. a metal tube) extending through the consumable 103. The passage 106 is in fluid communication with a gap defined between the consumable 103 and the main body 102 (when engaged) such that air outside of the system 101 is drawn into the passage 106 (during an inhale).

The smoking substitute system 101 is configured to vaporise the e-liquid 104 for inhalation by a user. To provide this, the consumable 103 comprises a heater having of a porous wick 110 and a resistive heating element in the form of a heating filament 111 that is helically wound around a portion of the porous wick 110. The porous wick 110 extends across the passage 106 (i.e. transverse to a longitudinal axis of the passage106) and opposing ends of the wick 110 extend into the tank 105 (so as to be submerged in the e-liquid 104). In this way, e-liquid 104 contained in the tank 105 is conveyed from the opposing ends of the porous wick 110 to a central portion of the porous wick 110 so as to be exposed to the airflow in the passage 106 (i.e. caused by a user inhaling). In other embodiments the heating filament 111 and/or wick 110 may form part of the main body (but may engage the tank 105 during engagement of the main body 102 and the consumable 103).

The helical filament 111 is wound about this exposed central portion of the porous wick 110 and is electrically connected to an electrical interface in the form of electrical contacts 112 mounted at the end of the consumable that is proximate the main body 102 (when engaged). When the consumable 103 is engaged with the main body 102, the electrical contacts 112 contact corresponding electrical contacts (not shown) of the main body 102. The main body electrical contacts are electrically connected to a power source (not shown) of the main body 102, such that (in the engaged position) the filament 111 is electrically connected to the power source. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow and, between the vaporisation point at the filament 111 and the outlet 108 of the passage 106, condenses to form an aerosol. This aerosol is then inhaled, via the mouthpiece 109, by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface.

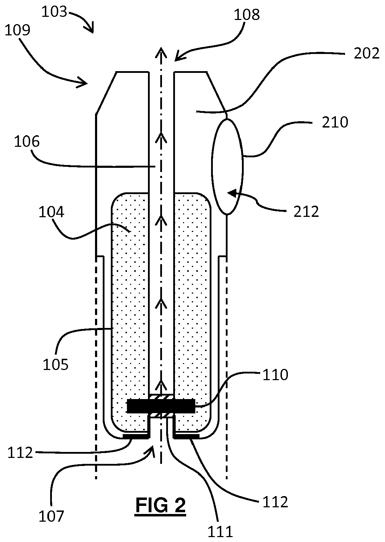

Figure 2 illustrates a consumable 103 for a smoking substitute device such as the electronic cigarette system shown in Figures 1A-C. The consumable 103 has an outer casing 202 in which the tank 105 containing the e-liquid 104 is disposed. The passage 106 is disposed through the outer casing 202.

A flavour delivery apparatus 210 is positioned in the outer casing 202. The flavour delivery apparatus 210 is a compressible pocket. The compressible pocket 210 is moulded integrally as part of the outer casing 202 so that at least part of the pocket extends outwardly from an outer surface of the outer casing 202. In Figure 2, a portion of the pocket is positioned inside the outer casing 202 of the consumable 103, and another portion of the pocket is positioned to extend away and outwardly from the outer casing 202 in an uncompressed state.

The compressible pocket 210 is distinct and separate from the tank 105 containing the e-liquid, and the passage 106 through the consumable 103. The compressible pocket is thus fluidly isolated from the tank 105 and the passage 106. Specifically, the flow path of the air from the inlet 107 to the outlet 108 of the passage 106 is thus separate and distinct from the pocket 210 so that the flow of air through the tank 105 does not come into contact with the compressible pocket 210 or the contents thereof.

The compressible pocket 210 has an internal volume defining a reservoir which contains a fragranced liquid 212. The fragranced liquid 212 is configured to release a fragrance, smell or odour, and may be oil-based and/or sugar-based. The compressible pocket may also contain air.

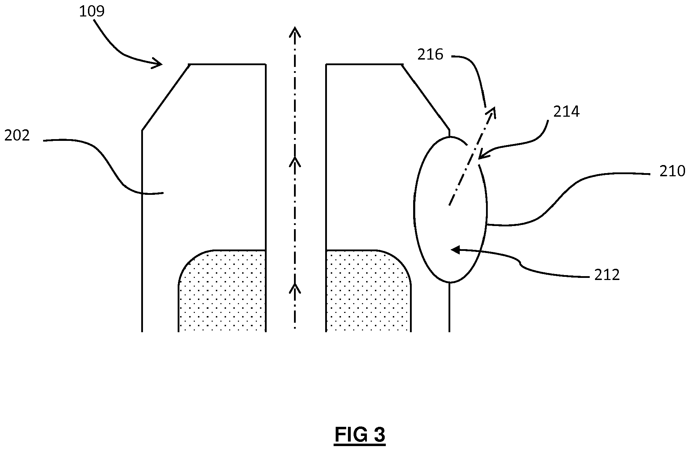

The pocket 210 has an outlet mister having a plurality of slits and/or holes. The outlet mister is formed from a silicon valve. The outlet mister is configured to form a mist of fragranced liquid 212 as the fragranced liquid is urged therethough, the resulting mist thus being released from the pocket 210 into the surrounding ambient air (as shown using the dashed arrow 216 in Figure 3). The pocket 210 also has a one-way valve inlet which allows surrounding ambient air into the pocket 210, but prevents air escaping from the pocket 210.

In Figure 3, the outlet mister is integral with the valve inlet, such that they form a single combined valve 214.

During compression of the pocket 210 from an uncompressed state (as shown in Figures 2 and 3) to a compressed state, the single combined valve 214 acts as the outlet mister. After the compression of the pocket 210, the single combined valve 214 acts as a one-way valve outlet which allows surrounding air into the pocket 210, but prevents air in the pocket 210 from escaping from the pocket. Thus, the pocket 210 can return from the compressed state to the uncompressed state after each compression of the pocket 210.

A user of the electronic cigarette device can actuate the release of flavour from the pocket 210 by compressing, or pushing, the outwardly directed portion of the pocket 210 towards the outer casing 202 of the consumable. In other words, the user compresses the pocket 210 from the uncompressed state to the compressed state. The movement of the pocket 210 from the uncompressed state to the compressed state acts to blow a combination of air and fragranced liquid 212 in the pocket 210 through the single combined valve 214. During compression, the single combined valve 214 acts as an outlet mister and creates a fine mist of droplets of the fragranced liquid 212 (hereinafter a fragranced vapour mist) which passes out of the single combined valve 214 and into the surrounding ambient air. The fragranced vapour mist can then travel towards a user's nose for olfactory flavour delivery.

After compression of the pocket 210, and when the user has stopped compressing the pocket 210, the single combined valve 214 acts as a one-way valve, allowing surrounding outside air into the pocket through the single combined valve 214, but preventing air already inside the pocket 210 from escaping from the pocket 210. As the single combined valve 214 acts as a one-way valve after compression of the pocket 210, the pocket 210 returns from the compressed state to the uncompressed state. The pocket 210 can then be compressed again by a user to repeatedly release fragranced vapour mist.

In an alternative embodiment, the one-way valve may be separate from the outlet mister.

The pocket 210 is positioned on an outside of the outer casing 202 of the consumable, such that a user can easily access the pocket 210 for compression with, for example, a finger in order to actuate flavour delivery.

The outlet mister (which in the embodiment shown in Figure 3 is the single combined valve 214) is positioned on the pocket 210 near an upper end of the pocket 210 adjacent to the mouthpiece 109 of the consumable 103. Therefore, in use (i.e. during vaping), the outlet mister is positioned underneath and/or adjacent to the nose of the user for olfactory flavour delivery.

In some embodiments, it is envisaged that the flavour delivery article may be detachable from the outer casing 202. For example, the flavour delivery article may be releasably clipped onto the outer casing 202 of the consumable 103 in order to permit convenient replacement and/or change the flavour delivery article. Therefore, a user can change the type of flavour (e.g. from a fruit flavour to a spiced flavour, for example), or can replace an empty flavour delivery article, in which all the fragranced liquid has been used. Alternatively, the flavour delivery article may be integral with or otherwise permanently attached to the outer casing 202, such that the flavour of the consumable may only be changed and/or the flavour delivery article replaced along with the replaceable consumable 103.

Alternatively and/or additionally, the flavour delivery article may be refillable with fragranced liquid such that the flavour delivery article is reusable.

In an alternative embodiment, the pocket is not attached to the consumable 103, but is instead attached to the main body 102 of the smoking substitute device.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A flavour delivery article for use with a smoking substitute device, wherein the flavour delivery article contains a fragranced fluid, and is configured to release a mist of the fragranced fluid upon actuation of the flavour delivery article for olfactory flavour delivery.

2. The flavour delivery article according to claim 1, wherein the flavour delivery article is configured to release the mist of the fragranced fluid only when the flavour delivery article is actuated, and is configured to not release the mist of the fragranced fluid when the flavour delivery article is not actuated.

3. The flavour delivery article according to claim 1 or claim 2, wherein the flavour delivery article is a compressible pocket, the compressible pocket is biased into an uncompressed state, and the compressible pocket is configured to release the mist of the fragranced fluid upon compression of the compressible pocket.

4. The flavour delivery article according to claim 3, further comprising a valve inlet for introducing ambient air into the pocket.

5. The flavour delivery article according to claim 4, wherein the valve inlet is a one-way valve configured to permit the flow of air through the valve inlet into the compressible pocket, and to prevent the flow of air through the valve inlet out of the compressible pocket.

6. The flavour delivery article according to any preceding claim, further comprising an outlet mister for releasing the mist of the fragranced fluid upon actuation of the flavour delivery article.

7. The flavour delivery article according to claim 6, wherein the outlet mister comprises a plurality of holes or slits for releasing the mist of the fragranced fluid.

8. The flavour delivery article according to claim 6 or claim 7, wherein the outlet mister is a silicone valve.

9. The flavour delivery article according to any preceding claim, wherein the fragranced fluid is an oil-based liquid and/or a sugar-based liquid.

10. A smoking substitute apparatus for a smoking substitute device, the smoking substitute apparatus comprising:
a tank for containing a vaporisable e-liquid;
a heating element for heating the vaporisable e-liquid; and
the flavour delivery article of any preceding claim.

11. The smoking substitute apparatus according to claim 10, further comprising an outer casing, wherein the flavour delivery article is moulded into the outer casing.

12. The smoking substitute apparatus according to claim 10 or claim 11 having opposed ends, wherein:
one end of the smoking substitute apparatus defines a mouthpiece;
the flavour delivery article further comprises an outlet mister for releasing the mist of the fragranced fluid upon actuation of the flavour delivery article; and
the outlet mister of the flavour delivery article is positioned such that is closer to the end of the smoking substitute apparatus defining the mouthpiece than it is to the other end of the smoking substitute apparatus.

13. The smoking substitute apparatus according to claim 12, wherein the outlet mister is configured to release the mist of the fragranced fluid towards the mouthpiece.

14. The smoking substitute apparatus according to any of claims 10-13, wherein the flavour delivery article is separated from the tank for containing the e-liquid.

15. A smoking substitute device comprising:
the smoking substitute apparatus of any of claims 10-14; and
a main body comprising a power source for providing power to the heating element.
